# DEMANDE DE BREVET EUROPEEN

(11) **EP 4 803 603 A1**
(43) Date de publication de la demande: **09.09.2026**
(21) Numéro de dépôt: 26161539.7
(22) Date de dépôt: 02.03.2026
(51) Int. Cl.: C12M 1/00, C12M 1/04

(54) **PHOTOBIORÉACTEUR, ADAPTÉ À LA CULTURE DE MICROALGUES**

(30) Priorité: 04.03.2025 FR 2502183
(71) Demandeur: Zeni, 44600 Saint-Nazaire (FR)
(72) Inventeur: TANGUY, Guillaume, 44000 NANTES (FR)
(74) Mandataire: Jacobacci & Partners France

(57) **Abrégé**

La présente invention concerne un photobioréacteur (10) comprenant une cuve de culture (20) adaptée à recevoir une culture de microalgues.

La cuve de culture (20) est équipée de moyens d'agitation (40), immergés, comprenant un rotor (41).

Ce rotor (41) comprend un moyeu (411) qui est destiné à être raccordé à une alimentation (50) en flux d'air et qui est prolongé par au moins un bras radial tubulaire (412) sur lequel sont répartis des orifices de bullage (4121) pour la libération dudit flux d'air.

Et au moins une partie de la longueur dudit au moins un bras radial tubulaire (412) épouse ladite paroi de fond (21) de sorte qu'une première cote en distance (L1) entre l'un au moins desdits orifices de bullage (4121) et ladite paroi de fond (21) est égale ou inférieure à 50 mm, de préférence de 5 mm à 20 mm.

## Description

### Domaine technique de l'invention

La présente invention concerne le domaine technique des photobioréacteurs, adaptés à la culture de microalgues.

### Etat de la technique

Les photobioréacteurs sont des dispositifs permettant la culture de microalgues en milieu contrôlé, avec une optimisation des paramètres tels que la lumière, le pH, la température, le gaz dissous et le mouvement du fluide.

Cependant, un des défis majeurs rencontrés dans ces systèmes est la formation de biofilm sur les parois internes de la cuve et les composants en contact avec la culture. Ce phénomène entraîne une diminution de la productivité, crée un environnement propice au développement des contaminations et peut mener à des crashs de culture.

Plus particulièrement, ce biofilm représente un risque important pour la culture de microalgues en réduisant l'accès à la lumière et en bloquant certains capteurs utilisés pour surveiller la culture.

En particulier, dans les photobioréacteurs à lumière immergée, la présence de biofilm sur les sources lumineuses altère considérablement la transmission de la lumière, limitant la croissance des microalgues et diminuant ainsi l'efficacité du procédé.

La formation de biofilm perturbe également l'écoulement du fluide en créant des zones de faible turbulence où les microalgues peuvent stagner et sédimenter, rendant certaines parties du réacteur inexploitables.

Ce phénomène nécessite en outre un nettoyage fréquent, impliquant généralement l'usage de produits chimiques ou des cycles prolongés d'arrêt de production. La maintenance devient alors un facteur contraignant qui réduit l'efficacité opérationnelle des photobioréacteurs.

Il existe ainsi un besoin d'une solution technique qui permettrait de réduire, voire de supprimer, ce phénomène de formation de biofilm dans les photobioréacteurs.

### Présentation de l'invention

Afin de remédier à l'inconvénient précité de l'état de la technique, la présente invention propose un photobioréacteur comprenant une cuve de culture adaptée à recevoir une culture de microalgues.

La cuve de culture comporte une paroi de fond prolongée par une paroi latérale définissant un axe longitudinal.

La cuve de culture est équipée :
- de moyens d'éclairage, immergés,
- de moyens d'agitation, immergés, comprenant un rotor apte à pivoter autour d'un axe de rotation orienté coaxialement par rapport audit axe longitudinal.

Et, selon l'invention, ledit rotor comprend un moyeu qui est destiné à être raccordé à une alimentation en flux d'air et qui est prolongé par au moins un bras radial tubulaire sur lequel sont répartis des orifices de bullage pour la libération dudit flux d'air.

Et au moins une partie de la longueur dudit au moins un bras radial tubulaire épouse ladite paroi de fond de sorte qu'une première cote en distance entre l'un au moins desdits orifices de bullage et ladite paroi de fond est égale ou inférieure à 50 mm, de préférence de 5 mm à 20 mm.

Il a été observé que cette solution technique assure une limitation, voire une suppression, du biofilm au niveau de la paroi de fond.

Le passage du rotor empêche la sédimentation et la formation de biofilm, qui est une source de contaminants biologiques, prédateurs ou pathogènes de microalgues.

Selon d'autres caractéristiques techniques avantageuses, pouvant être prises en combinaison ou indépendamment :
- la paroi de fond définit une surface projetée totale, et la surface projetée dudit au moins un bras radial tubulaire sur ladite paroi de fond est inférieure à 25% de ladite surface projetée totale de la paroi de fond ;
- chaque orifice de bullage s'inscrit dans une trajectoire circulaire, et la trajectoire circulaire d'au moins un orifice de bullage est sous-jacente (selon une direction parallèle à l'axe longitudinal) d'au moins un élément immergé qui est susceptible d'être recouvert par un biofilm, ledit au moins un élément immergé est choisi parmi les moyens d'éclairage, les crosses de régulation thermique ou des moyens de mesure d'un paramètre de la culture ; de préférence, la trajectoire circulaire de l'un au moins desdits orifices de bullage définit une deuxième cote en distance qui est égale ou inférieure à 50 mm, de préférence 20 mm, par rapport audit au moins un élément immergé ;
- chaque orifice de bullage s'inscrit dans une trajectoire circulaire, et ladite trajectoire circulaire de l'un au moins desdits orifices de bullage définit une troisième cote en distance qui est égale ou inférieure à 50 mm, de préférence 20 mm, par rapport à la paroi latérale ;
- le moyeu est porté par un stator qui est prolongé par un conduit d'admission, lequel conduit d'admission traverse la paroi de fond ou une paroi supérieure ; de préférence, ledit moyeu et ledit stator sont assemblés par des moyens d'assemblage en rotation, formant avantageusement un pallier lisse et avantageusement dépourvus de joint ou de roulement rapporté ; de préférence encore, le stator comporte une partie tubulaire cylindrique comportant une surface périphérique cylindrique terminée par une surface supérieure et par un épaulement annulaire inférieur, et le moyeu comporte un corps prolongé par ledit au moins un bras radial tubulaire et une jupe emmanchée sur ladite partie tubulaire cylindrique et verrouillée en translation par une bague coopérant avec ledit épaulement annulaire inférieur ;
- la paroi de fond présente une forme générale tronconique, concave, et ledit au moins un bras radial tubulaire épouse ladite paroi de fond ;
- ledit rotor comporte au moins deux bras radiaux tubulaires, avantageusement répartis régulièrement autour dudit moyeu ;
- les orifices de bullage sont ménagés de sorte à générer une rotation dudit rotor en présence du flux d'air ;
- les orifices de bullage présentent un diamètre allant de 0,5 mm à 2 mm ;
- l'un au moins des orifices de bullage se situe à une distance maximale de 20 mm par rapport à une extrémité libre du bras radial tubulaire, ladite extrémité étant elle-même située à une distance maximale de 30 mm de la paroi latérale de la cuve de culture ;
- sur un bras radial tubulaire, les orifices de bullage sont espacés les uns des autres d'une distance allant de 30 mm à 100 mm.

La présente invention concerne encore le procédé de culture de microalgues par la mise en œuvre d'un photobioréacteur selon l'invention.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

De plus, diverses autres caractéristiques de l'invention ressortent de la description annexée effectuée en référence aux dessins qui illustrent des formes, non limitatives, de réalisation de l'invention et où :
[Fig. 1] est une vue schématique, en coupe, d'un photobioréacteur selon l'invention ;
[Fig. 2] est une vue schématique, vue de dessus, d'un rotor équipant le photobioréacteur selon l'invention ;
[Fig. 3] est une vue schématique, vue de côté, d'un mode de réalisation pour un rotor selon l'invention ;
[Fig. 4] est une vue schématique, selon un plan de coupe, du rotor selon la figure 3 ;
[Fig. 5] est une vue schématique, partielle et agrandie, du rotor selon les figures 3 ou 4.

Il est à noter que, sur ces figures, les éléments structurels et/ou fonctionnels communs aux différentes variantes peuvent présenter les mêmes références.

La présente invention concerne ainsi un photobioréacteur 10, adapté à recevoir une culture de microalgues.

Par « photobioréacteur », on entend avantageusement un réacteur biologique conçu pour la culture de micro-organismes photosynthétiques, en l'espèce de microalgues. Tel que développé ci-après, ce réacteur est équipé de moyens permettant d'optimiser l'apport en lumière, en nutriments et en gaz afin de favoriser la croissance et la productivité des organismes cultivés.

Les microalgues sont des micro-organismes aquatiques, généralement unicellulaires ou formant des structures simples, capables de réaliser la photosynthèse.

Elles appartiennent à des groupes très diversifiés, incluant :
- des eucaryotes, comme les algues vertes et rouges, et
- des procaryotes, comme les cyanobactéries, également appelées algues bleues.

De manière générale, le photobioréacteur 10 selon l'invention comprend une cuve de culture 20 adaptée à recevoir la culture de microalgues.

Et, selon l'invention, la cuve de culture 20 est équipée d'éléments immergés :
- de moyens d'éclairage 30, immergés,
- de moyens d'agitation 40, immergés, comprenant un rotor 41, et éventuellement
- des moyens de mesure 80 d'un paramètre de la culture (non représentés), par exemple un capteur de pH, de température, de gaz, ou un capteur optique.

Tel que décrit ci-après et selon l'invention, le rotor 41 forme un bulleur rotatif conçu pour limiter, voire éliminer, la formation de biofilm au sein de la cuve de culture 20.

En effet, le rotor 41 constitue un bulleur rotatif spécialement conçu pour générer un mouvement fluide et homogène au sein de la cuve de culture 20. Grâce à sa structure optimisée et à la dynamique de bullage induite par l'éjection d'air, ce dispositif permet de limiter significativement l'adhésion des micro-organismes aux surfaces internes du réacteur, réduisant ainsi la formation de biofilm.

### Cuve de culture

Par « cuve de culture », on entend avantageusement un réservoir conçu pour accueillir et maintenir un milieu de culture liquide dans lequel se développent les microalgues.

La cuve de culture 20 comporte une paroi de fond 21 prolongée par une paroi latérale 22 définissant un axe longitudinal 20'. Cette paroi latérale 22 est avantageusement terminée par une paroi supérieure 23.

Par « paroi de fond », on entend avantageusement la paroi inférieure délimitant la cuve de culture.

De manière non limitative, la paroi de fond 21 est ici tronconique ou conique, dont la surface supérieure est concave.

Par « paroi latérale 22 », on entend avantageusement la surface verticale ou approximativement verticale délimitant la cuve de culture 20, s'étendant depuis la paroi de fond jusqu'à la partie supérieure du photobioréacteur.

La paroi latérale 22 peut être conçue avec différentes géométries, en fonction des exigences du réacteur, par exemple cylindrique.

### Moyens d'éclairage

Par « moyens d'éclairage », on entend avantageusement un dispositif lumineux placé à l'intérieur de la cuve de culture 20 et conçu pour fournir un apport lumineux direct et homogène aux microalgues en culture.

Ces moyens d'éclairage sont entièrement ou partiellement immergés dans le milieu de culture afin d'optimiser l'absorption de la lumière par les organismes photosynthétiques et d'améliorer l'efficacité du processus de croissance.

Les moyens d'éclairage 30 peuvent être constitués de sources lumineuses artificielles adaptées aux besoins spécifiques des microalgues, notamment des diodes électroluminescentes (LEDs) immergées.

Ces moyens d'éclairage 30 comprennent avantageusement plusieurs éléments lumineux, répartis autour et parallèlement à l'axe longitudinal 20'.

Dans le cadre de la présente invention et tel que développé ci-après, les moyens d'éclairage 30 immergés sont avantageusement positionnés de manière à minimiser la formation de biofilm sur leur surface, notamment en étant placés dans des zones où le bullage généré par le rotor assure un auto-nettoyage efficace.

Une telle configuration vise à éviter l'obstruction progressive de la lumière par des dépôts biologiques, garantissant ainsi une transmission lumineuse constante et une productivité optimale du système.

### Moyens d'agitation

Selon l'invention, le rotor 41 apte à pivoter autour d'un axe de rotation 41' orienté coaxialement par rapport à l'axe longitudinal 20'.

Par exemple, la vitesse de rotation est comprise en 4 tours/min et 20 tours/min.

Encore selon l'invention, ce rotor 41 comprend un moyeu 411 qui est prolongé par au moins un bras radial tubulaire 412. Le moyeu 411 est destiné à être raccordé à une alimentation 50 en flux d'air.

Par « alimentation en flux d'air », on entend avantageusement un dispositif conçu pour fournir un apport contrôlé en air ou en gaz, permettant ainsi d'assurer l'oxygénation du milieu, le mélange des microalgues et la régulation des échanges gazeux.

En particulier, par « moyeu », on entend avantageusement un élément central du rotor 41, destiné à être raccordé à l'alimentation 50 en flux d'air et à assurer la distribution homogène de l'air vers ledit au moins un bras tubulaire 412.

Pour cela, le moyeu 41 comporte avantageusement des conduits de gaz convenablement structurés pour assurer le raccordement gazeux entre l'alimentation 50 et ledit au moins un bras radial tubulaire 412.

Par ailleurs, le moyeu 411 est prolongé par un bras radial tubulaire 412, voire de préférence par une pluralité de bras radiaux tubulaires 412.

La rotation dudit au moins un bras radial tubulaire 412 permet de balayer la circonférence de la paroi de fond 21, en assurant une agitation douce.

De préférence, le rotor 41 comporte au moins deux bras radiaux tubulaires 412, avantageusement répartis régulièrement autour du moyeu 411.

Par « bras radial tubulaire », on entend avantageusement un élément allongé et creux, s'étendant radialement à partir du moyeu 411 du rotor 41 et conçu pour acheminer et distribuer le flux d'air.

Pour cela, ledit au moins un bras radial tubulaire 412 comporte des orifices de bullage 4121 pour la libération du flux d'air.

Par « orifices de bullage 4121 », on entend avantageusement des ouvertures traversantes qui sont ménagées sur ledit au moins un bras radial tubulaire du rotor 41, permettant la libération contrôlée du flux d'air à l'intérieur de la cuve de culture 20.

Par exemple, les orifices de bullage 4121 présentent un diamètre allant de 0,5 mm à 2 mm.

Ces orifices sont conçus pour générer un bullage efficace, assurant l'agitation du fluide, l'oxygénation du milieu et la prévention de l'accumulation de biofilm sur les surfaces internes de la cuve.

Pour cela, ces orifices de bullage 4121 sont répartis sur ledit au moins un bras radial tubulaire 412, avantageusement sur sa longueur.

Par exemple, sur un bras radial tubulaire 412, les orifices de bullage 4121 sont espacés les uns des autres d'une distance allant de 30 mm à 100 mm.

Encore selon l'invention, au moins une partie de la longueur dudit au moins un bras radial tubulaire 412 épouse la paroi de fond 21.

En d'autres termes, le bras radial tubulaire 412 est conçu de manière à suivre, sur une portion déterminée de sa longueur, la géométrie de la paroi de fond 21, en maintenant une distance contrôlée et optimisée par rapport à celle-ci.

Plus précisément, la première cote en distance L1 entre l'un au moins des orifices de bullage 4121 et la paroi de fond 21 est égale ou inférieure à 50 mm, de préférence de 5 mm à 20 mm.

Par « cote en distance », on entend avantageusement la distance minimale séparant un orifice de bullage situé sur un bras radial tubulaire du rotor 41 et la surface interne de la paroi de fond 21 de la cuve de culture 20.

De préférence, cette distance correspond à l'écart le plus court entre l'orifice de bullage et la paroi de fond. Une optimisation de cette distance permet de maximiser l'interaction des bulles d'air avec la paroi de fond afin de limiter l'accumulation de dépôts, d'empêcher la formation de biofilm et d'améliorer l'homogénéisation du milieu de culture.

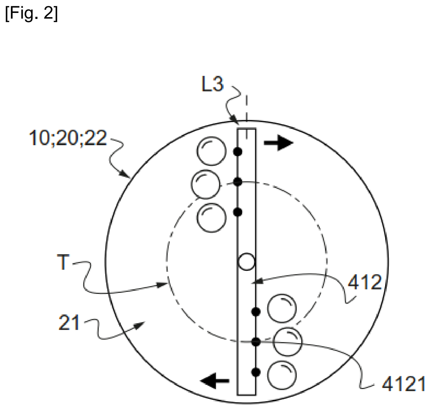

Toujours selon l'invention, chaque orifice de bullage 4121 s'inscrit dans une trajectoire circulaire T (voir la figure 2).

Les orifices de bullage 4121, positionnés sur le bras radial tubulaire 412, suit ainsi un chemin circulaire lorsque le rotor 41 est en rotation, coaxialement à l'axe de rotation 41'.

Et la trajectoire circulaire T d'au moins un orifice de bullage 4121 est sous-jacente (selon une direction D parallèle à l'axe longitudinal 20') d'au moins un élément immergé qui est susceptible d'être recouvert par un biofilm.

Ledit au moins un élément immergé est choisi parmi les moyens d'éclairage 30 les crosses de régulation thermique ou des moyens de mesure 80 d'un paramètre de la culture.

Par « crosses de régulation thermique », on entend avantageusement des éléments structuraux conçus pour assurer une gestion thermique efficace au sein d'un dispositif, en facilitant l'échange de chaleur entre différentes zones du système.

En d'autres termes, un orifice de bullage 4121 suit une trajectoire située sous un élément immergé, lorsque l'on considère une direction D parallèle à l'axe longitudinal 20'.

Sans être limité par une quelconque théorie, le flux de bulles généré par cet orifice de bullage 4121 remonte directement sous et le long de cet élément immergé, favorisant ainsi son contact avec l'air injecté et réduisant l'adhésion de dépôts biologiques susceptibles de former un biofilm.

Pour optimiser encore ce phénomène, la trajectoire circulaire T de l'un au moins desdits orifices de bullage 4121 définit une deuxième cote en distance L2 qui est égale ou inférieure à 50 mm, de préférence 20 mm, par rapport audit au moins un élément immergé.

De même, la trajectoire circulaire T de l'un au moins desdits orifices de bullage 4121 définit une troisième cote en distance L3 qui est égale ou inférieure à 50 mm, de préférence 20 mm, par rapport à la paroi latérale 22.

De préférence encore, l'un au moins des orifices de bullage 4121 se situe à une distance maximale de 20 mm par rapport à une extrémité libre 412a du bras radial tubulaire 412. Cette extrémité est elle-même située à une distance maximale de 30 mm de la paroi latérale 22 de la cuve de culture 20.

Sans être limité par une quelconque théorie, le flux de bulles généré par cet orifice de bullage 4121 remonte directement sous et le long de la paroi latérale 22, favorisant ainsi son contact avec l'air injecté et réduisant l'adhésion de dépôts biologiques susceptibles de former un biofilm.

Selon une caractéristique avantageuse, les orifices de bullage 4121 sont ménagés de sorte à générer une rotation du rotor 41 en présence du flux d'air.

En d'autres termes, les orifices de bullage 4121 sont disposés de manière à générer une rotation du rotor 41 sous l'effet du flux d'air. Cette configuration permet d'exploiter l'effet de poussée exercé par les bulles d'air, créant ainsi un couple de rotation qui entraîne le mouvement du rotor 41.

Plus précisément, ces orifices de bullage 4121 sont avantageusement disposés sur un bras radial tubulaire 412 de manière opposée au sens de rotation recherché, de manière à exploiter l'effet de réaction du flux d'air éjecté.

Ainsi, lorsque l'air est expulsé par les orifices de bullage 4121, la poussée générée crée un couple de force qui entraîne la mise en mouvement du rotor 41.

L'entraînement du rotor est entièrement assuré par l'injection d'air, supprimant le besoin d'un moteur additionnel et simplifiant la conception du système.

Le système de bullage rotatif est ainsi avantageusement du type « autoamorçant ». Ce dispositif fonctionne sans moteur, utilisant l'éjection d'air à travers des orifices positionnés stratégiquement sur ses bras pour créer un mouvement rotatif. Cette rotation permet également de balayer efficacement les surfaces internes du photobioréacteur, empêchant ainsi l'accumulation de biofilm.

De préférence, la forme de la section du bras radial tubulaire 412 est hydrodynamique, optimisée pour limiter la traînée et favoriser l'écoulement fluide du milieu environnant. Elle peut être circulaire ou présenter un profil avec un intrados et un extrados, ou adopter une forme en goutte d'eau, avec les orifices de bullage idéalement disposés sur le bord le plus large afin d'optimiser la dispersion des bulles et de minimiser les perturbations hydrodynamiques.

En outre, ledit au moins un bras radial tubulaire 412 présente avantageusement une dimension spécifique.

A cet égard, la paroi de fond 21 définit une surface projetée totale. Par « surface projetée totale », on entend avantageusement la surface de la paroi de fond 21, lorsqu'elle est projetée dans un plan horizontal. Par exemple, dans le cas d'une paroi de fond circulaire ou tronconique, la surface projetée totale correspond à la surface de la paroi de fond 21.

Et ledit au moins un bras radial tubulaire 412 définit une surface projetée sur ladite paroi de fond 21.

Cette « surface projetée » correspond avantageusement l'empreinte bidimensionnelle résultant de la projection orthogonale d'au moins un bras radial tubulaire 412 sur la paroi de fond 21.

Et cette surface projetée, sur ladite paroi de fond 21, dudit au moins un bras radial tubulaire 412 est inférieure à 25 % de cette surface projetée totale.

Encore selon l'invention, la structure de l'admission d'air vers le moyeu 411 comporte ici des caractéristiques techniques avantageuses.

A cet égard, le moyeu 411 est avantageusement porté par un stator 60 qui est prolongé par un conduit d'admission 61.

En d'autres termes, le photobioréacteur 10 comporte ainsi avantageusement une conduite pour l'alimentation en gaz qui comporte :
- un tronçon amont, statique, comprenant le conduit d'admission 61 terminé par le stator 60, et
- un tronçon aval, rotatif, s'étendant le long du rotor 41, et plus précisément au sein du moyeu 411 puis dudit au moins un bras radial tubulaire 412.

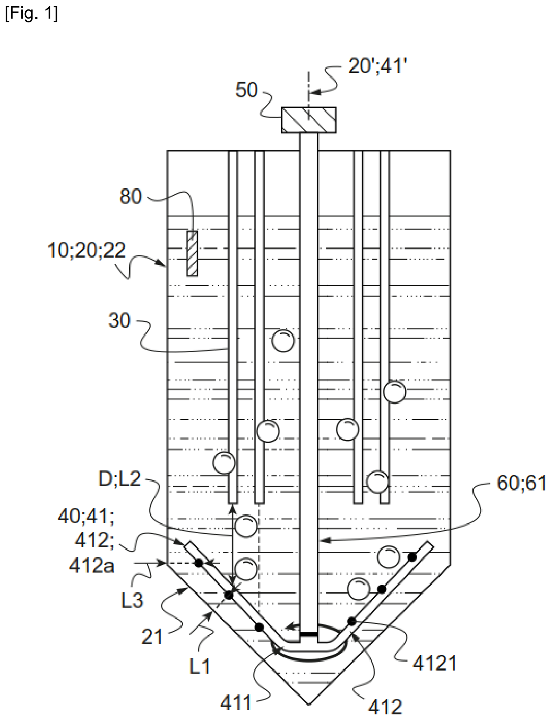

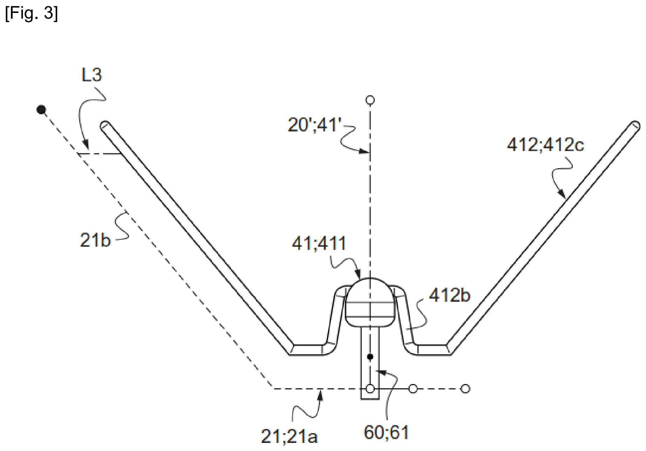

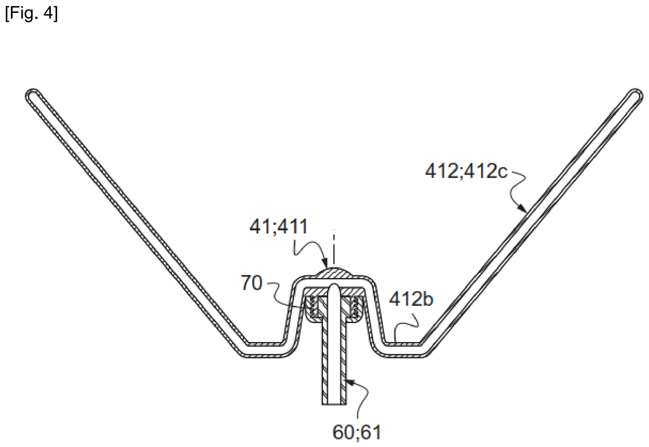

Ce conduit d'admission 61, avantageusement statique, comporte avantageusement plusieurs possibilités d'agencement :
- le conduit d'admission 61 traverse la paroi de fond 21 (figures 3 à 5), ou
- le conduit d'admission 61 traverse une paroi supérieure 23 (figure 1).

Ce conduit d'admission 61, statique et s'étendant partiellement dans la cuve de culture 20, évite l'utilisation d'un joint tournant entre ce conduit d'admission 61 et la paroi 21, 23 correspondante.

### Mode de réalisation particulier

Les figures 3 à 5 illustrent un mode de réalisation particulier de l'invention.

En particulier, le moyeu 411 et le stator 60 sont assemblés par des moyens d'assemblage en rotation 70.

Ces moyens d'assemblage en rotation 70 forment avantageusement un pallier lisse, qui est avantageusement dépourvus de joint rapporté.

Par « palier lisse », on entend avantageusement un dispositif d'assemblage en rotation permettant un mouvement fluide entre le moyeu 411 et le stator 60, sans recours à des éléments roulants (tels que des roulements à billes).

Les surfaces en contact peuvent avantageusement être équipées d'un élément destiné à réduire le frottement, tel qu'une pièce en PTFE (polytétrafluoroéthylène) ou tout autre matériau à faible coefficient de friction. Cet élément permet de minimiser l'usure mécanique, faciliter le mouvement relatif des pièces et améliorer la durabilité du système.

Cette configuration :
- optimise la fluidité de la rotation du rotor 41, avantageusement entraîné par le flux d'air,
- améliore la longévité du système en réduisant les contraintes mécaniques.

Par « dépourvus de joint rapporté », on entend avantageusement une conception mécanique dans laquelle le moyeu 411 et le stator 60, en mouvement relatif, ne nécessitent pas de joint d'étanchéité additionnel pour assurer le circulation du flux d'air, ou inversement un phénomène d'infiltration.

Le système est ainsi avantageusement conçu sans roulement, ce qui simplifie considérablement la maintenance et réduit les risques de défaillance mécanique.

Sans être limité par une quelconque théorie, le mouvement circulaire est obtenu avec un faible frottement (résultant d'un jeu suffisant) entre les pièces. La présence d'un coussin d'air entre les pièces participe encore à l'étanchéité.

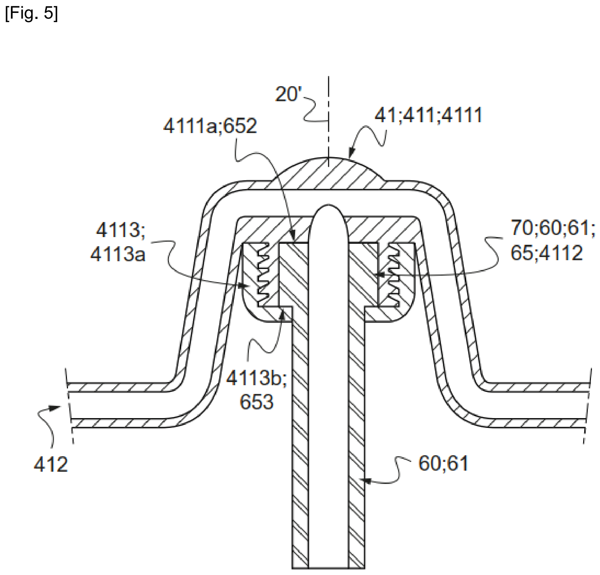

Selon le présent mode de réalisation, tel que représenté plus en détails sur la figure 5, le stator 60 comporte une partie tubulaire cylindrique 65, formant une collerette, qui est avantageusement ménagée au niveau de l'extrémité du conduit d'admission 61.

Cette partie tubulaire cylindrique 65 comporte une surface périphérique cylindrique 651 terminée par :
- une surface supérieure 652, libre et en forme de couronne, et
- un épaulement annulaire inférieur 653.

La surface périphérique cylindrique 651 est avantageusement coaxiale avec l'axe longitudinal 20'.

La surface supérieure 652 et l'épaulement annulaire inférieure 653 sont avantageusement parallèles l'un par rapport à l'autre.

Et le moyeu 411 comporte quant à lui un corps 4111 prolongé par :
- ledit au moins un bras radial tubulaire 412, et
- une jupe 4112 emmanchée sur la partie tubulaire cylindrique 65.

Le corps 4111 comporte ici une surface d'appui 4111a, complémentaire de la surface supérieure 652 de la partie tubulaire cylindrique 65.

La jupe 4112 est verrouillée en translation, sur cette partie tubulaire cylindrique 65, par une bague 4113 coopérant avec l'épaulement annulaire inférieur 653.

En particulier, la bague 4113 comporte avantageusement :
- une portion tubulaire 4113a (par exemple femelle), destinée à coopérer avec la jupe 4112 (par exemple mâle), par exemple par vissage, et
- une portion collerette 4113b, orientée pour former une surface d'appui sous l'épaulement annulaire inférieure 653.

En l'espèce, la partie tubulaire cylindrique 65 est ainsi enveloppée de manière étanche par le moyeu 411, tout en préservant un degré de liberté en rotation, dans lequel :
- la surface d'appui 4111a du corps 4111 est complémentaire de la surface supérieure 652 de la partie tubulaire cylindrique 65,
- la surface intérieure de la jupe 4112 est complémentaire de la partie tubulaire cylindrique 65, et
- la portion collerette 4113b est complémentaire de l'épaulement annulaire inférieure 653.

Par ailleurs, dans le présent mode de réalisation, la paroi de fond 21 présente une forme générale tronconique, concave.

De préférence, cette paroi de fond 21 comprend :
- une portion centrale 21a, en forme de disque, et
- une portion périphérique 21b, conique.

Et ledit au moins un bras radial tubulaire 412 épouse cette paroi de fond 21.

En l'espèce, le rotor 41 comporte ici au moins deux bras radiaux tubulaires 412 412 présente une forme générale de V, s'évasant du bas vers le haut.

Plus précisément encore, chaque bras radial tubulaire 412 comporte deux portions :
- une portion amont 412b, en L, épousant la portion centrale 21a, et
- une portion aval 412c, inclinée, épousant la portion périphérique 21b.

### Procédé de culture de microalgues

Le procédé de culture de microalgues selon l'invention repose sur la mise en œuvre du photobioréacteur 10, spécifiquement conçu pour optimiser la croissance des microalgues en minimisant les contraintes liées à la formation de biofilm et en assurant une oxygénation et une agitation efficaces du milieu de culture.

De manière avantageuse, le procédé commence par la préparation de la cuve de culture 20, dans laquelle est introduit un milieu nutritif adapté aux besoins des microalgues.

Une inoculation de microalgues est réalisée en introduisant une souche sélectionnée dans la cuve.

Le photobioréacteur 10 est équipé de moyens d'éclairage 30 immergés, optimisés pour fournir aux microalgues une intensité lumineuse homogène.

En pratique, ces moyens d'éclairage sont avantageusement positionnés de manière à éviter la formation de biofilm sur leur surface, notamment grâce à l'action du bullage.

L'agitation et l'oxygénation du milieu sont assurées par le rotor 41 dont ledit au moins un bras radial tubulaire 412 comporte les orifices de bullage 4121.

Ces orifices de bullage 4121 sont avantageusement agencés pour :
- générer un flux d'air assurant une oxygénation optimale du milieu,
- limiter la formation de biofilm sur les surfaces internes du réacteur, et
- de préférence, induire la rotation du rotor 41, améliorant ainsi le mélange des microalgues et des nutriments,

Le photobioréacteur 10 peut être équipé de moyens de mesure 80 permettant un suivi en temps réel des paramètres critiques, par exemple pH, température ou concentration en gaz.

Les valeurs mesurées permettent d'ajuster automatiquement l'injection de CO₂ ou l'intensité du bullage, garantissant ainsi des conditions optimales de culture.

La culture se poursuit jusqu'à atteindre une densité optimale de microalgues. A ce stade, différentes méthodes de récolte peuvent être employées (centrifugation, filtration ou floculation par exemple).

De manière générale, l'un des principaux avantages de l'invention réside dans la réduction du biofilm sur les surfaces immergées ; le rendement photosynthétique des microalgues reste maximal, augmentant ainsi l'efficacité globale du réacteur.

## Revendications

1. Photobioréacteur (10) comprenant une cuve de culture (20) adaptée à recevoir une culture de microalgues,
laquelle cuve de culture (20) comporte une paroi de fond (21) prolongée par une paroi latérale (22) définissant un axe longitudinal (20'),
laquelle cuve de culture (20) est équipée :
- de moyens d'éclairage (30), immergés,
- de moyens d'agitation (40), immergés, comprenant un rotor (41) apte à pivoter autour d'un axe de rotation (41') orienté coaxialement par rapport audit axe longitudinal (20'),
**caractérisé en ce que** ledit rotor (41) comprend un moyeu (411) qui est destiné à être raccordé à une alimentation (50) en flux d'air et qui est prolongé par au moins un bras radial tubulaire (412) sur lequel sont répartis des orifices de bullage (4121) pour la libération dudit flux d'air,
et **en ce qu'**au moins une partie de la longueur dudit au moins un bras radial tubulaire (412) épouse ladite paroi de fond (21) de sorte qu'une première cote en distance (L1) entre l'un au moins desdits orifices de bullage (4121) et ladite paroi de fond (21) est égale ou inférieure à 50 mm, de préférence de 5 mm à 20 mm.

2. Photobioréacteur (10) selon la revendication 1, **caractérisé en ce que** la paroi de fond (21) définit une surface projetée totale,
et **en ce que** la surface projetée dudit au moins un bras radial tubulaire (412) sur ladite paroi de fond (21) est inférieure à 25% de ladite surface projetée totale de la paroi de fond (21).

3. Photobioréacteur (10) selon l'une quelconque des revendications 1 ou 2, **caractérisé en ce que** chaque orifice de bullage (4121) s'inscrit dans une trajectoire circulaire (T),
et **en ce que** la trajectoire circulaire (T) d'au moins un orifice de bullage (4121) est sous-jacente d'au moins un élément immergé qui est susceptible d'être recouvert par un biofilm,
ledit au moins un élément immergé est choisi parmi les moyens d'éclairage (30), les crosses de régulation thermique ou des moyens de mesure (80) d'un paramètre de la culture.

4. Photobioréacteur (10) selon la revendication 3, **caractérisé en ce que** la trajectoire circulaire (T) de l'un au moins desdits orifices de bullage (4121) définit une deuxième cote en distance (L2) qui est égale ou inférieure à 50 mm, de préférence 20 mm, par rapport audit au moins un élément immergé.

5. Photobioréacteur (10) selon l'une quelconque des revendications 1 à 4, **caractérisé en ce que** chaque orifice de bullage (4121) s'inscrit dans une trajectoire circulaire (T),
et **en ce que** ladite trajectoire circulaire (T) de l'un au moins desdits orifices de bullage (4121) définit une troisième cote en distance (L3) qui est égale ou inférieure à 50 mm, de préférence 20 mm, par rapport à la paroi latérale (22).

6. Photobioréacteur (10) selon l'une quelconque des revendications 1 à 5, **caractérisé en ce que** le moyeu (411) est porté par un stator (60) qui est prolongé par un conduit d'admission (61),
lequel conduit d'admission (61) traverse la paroi de fond (21) ou une paroi supérieure (23).

7. Photobioréacteur (10) selon la revendication 6, **caractérisé en ce que** ledit moyeu (411) et ledit stator (60) sont assemblés par des moyens d'assemblage en rotation (70), formant avantageusement un pallier lisse et avantageusement dépourvus de joint rapporté.

8. Photobioréacteur (10) selon l'une quelconque des revendications 6 ou 7, **caractérisé en ce que** le stator (60) comporte une partie tubulaire cylindrique (65) comportant une surface périphérique cylindrique (651) terminée par une surface supérieure (652) et par un épaulement annulaire inférieur (653),
et **en ce que** le moyeu (411) comporte un corps (4111) prolongé par:
- ledit au moins un bras radial tubulaire (412) et
- une jupe (4112) emmanchée sur ladite partie tubulaire cylindrique (65) et verrouillée en translation par une bague (4113) coopérant avec ledit épaulement annulaire inférieur (653).

9. Photobioréacteur (10) selon l'une quelconque des revendications 1 à 8, **caractérisé en ce que** la paroi de fond (21) présente une forme générale tronconique, concave, et **en ce que** ledit au moins un bras radial tubulaire (412) épouse ladite paroi de fond (21).

10. Photobioréacteur (10) selon l'une quelconque des revendications 1 à 9, **caractérisé en ce que** ledit rotor (41) comporte au moins deux bras radiaux tubulaires (412), avantageusement répartis régulièrement autour dudit moyeu (411).

11. Photobioréacteur (10) selon l'une quelconque des revendications 1 à 10, **caractérisé en ce que** les orifices de bullage (4121) sont ménagés de sorte à générer une rotation dudit rotor (41) en présence du flux d'air.

12. Photobioréacteur (10) selon l'une quelconque des revendications 1 à 11, **caractérisé en ce que** l'un au moins des orifices de bullage (4121) se situe à une distance maximale de 20 mm par rapport à une extrémité libre du bras radial tubulaire (412), ladite extrémité étant elle-même située à une distance maximale de 30 mm de la paroi latérale (22) de la cuve de culture (20).

13. Procédé de culture de microalgues par la mise en œuvre d'un photobioréacteur (10) selon l'une quelconque des revendications 1 à 12.
